(19)

Europäisches
Patentamt

European
Patent Office

Office européen
des brevets

(11) **EP 1 818 015 B1**

(12) **FASCICULE DE BREVET EUROPEEN**

(45) Date de publication et mention
de la délivrance du brevet:
**08.06.2011 Bulletin 2011/23**

(51) Int Cl.:
*A61B 6/00* (2006.01)    *G01N 23/04* (2006.01)

(21) Numéro de dépôt: **07101862.6**

(22) Date de dépôt: **07.02.2007**

(54) **Procédé d'estimation du rayonnement diffusé en tomograpie par rayons X**

Verfahren zur Schätzung der Streustrahlung bei einer Röntgentomographie

Method of estimating scattered radiation in X-ray tomography

(84) Etats contractants désignés:
**DE GB IT**

(30) Priorité: **10.02.2006 FR 0650489**

(43) Date de publication de la demande:
**15.08.2007 Bulletin 2007/33**

(73) Titulaire: **Commissariat à l'Énergie Atomique
et aux Énergies Alternatives
75015 Paris (FR)**

(72) Inventeurs:
• **Rinkel, Jean
94158 San Francisco (US)**
• **Dinten, Jean-Marc
69008, LYON (FR)**

(74) Mandataire: **Poulin, Gérard et al
BREVALEX
95 rue d'Amsterdam
75378 Paris Cedex 8 (FR)**

(56) Documents cités:
**EP-A- 1 566 771    FR-A- 2 843 802**

• **CHUANG ET AL: "Novel scatter correction for
three-dimensional positron emission
tomography by use of a beam stopper device"
NUCLEAR INSTRUMENTS & METHODS IN
PHYSICS RESEARCH, SECTION - A:
ACCELERATORS, SPECTROMETERS,
DETECTORS AND ASSOCIATED EQUIPMENT,
ELSEVIER, AMSTERDAM, NL, vol. 551, no. 2-3,
11 octobre 2005 (2005-10-11), pages 540-552,
XP005095126 ISSN: 0168-9002**
• **OZARD S ET AL: "Monte Carlo validation of a
portal imager scatter dose model"
PROCEEDINGS OF THE 22ND ANNUAL
INTERNATIONAL CONFERENCE OF THE IEEE
ENGINEERING IN MEDICINE AND BIOLOGY
SOCIETY (CAT. NO.00CH37143) IEEE
PISCATAWAY, NJ, USA, vol. 4, 2000, pages
2889-2891 vol., XP002410478 ISBN:
0-7803-6465-1**

## Description

Le sujet de cette invention est un procédé d'estimation du rayonnement diffusé en tomographie par rayons X.

De nombreuses méthodes d'imagerie d'objets sont fondées sur l'atténuation subie par un rayonnement, et qui dépend de la nature de chaque portion des objets. Les rayons se projettent sur un détecteur bidimensionnel placé derrière l'objet. L'image tridimensionnelle est obtenue en procédant à plusieurs irradiations de l'objet à des angles différents d'incidence afin de disposer d'une quantité suffisante de mesures, et en recourant à un algorithme appelé d'inversion pour passer de la série d'images bidimensionnelles prises par le détecteur à l'image tridimensionnelle recherchée.

Or chaque pixel du détecteur reçoit, outre un rayon primaire projeté à travers l'objet, un rayonnement diffusé par l'objet et qui trouble la mesure. Le rayonnement diffusé, dont l'intensité est souvent importante, doit être estimé et corrigé pour améliorer la représentation de l'objet.

Plusieurs procédés ont été utilisés pour cela. Dans l'un d'entre eux, on a recours à un engin absorbeur du rayonnement composé d'un réseau de petites billes ("beam-stops") séparées de distances connues. Les rayons qui arrivent devant les billes sont entièrement absorbés, les autres passent sans atténuation. Quand on place à la fois l'objet à étudier et l'absorbeur entre la source du rayonnement et le détecteur bidimensionnel, les régions du détecteur qui sont situées derrière les billes forment des taches d'ombre illuminées seulement par le rayonnement diffusé par l'objet. On en déduit, par des interpolations entre les taches d'ombre, l'image du rayonnement diffusé sur toute la surface du détecteur. Cette image est soustraite de l'image ordinaire, prise en l'absence de l'absorbeur, de l'objet étudié. Le procédé donne de bons résultats mais nécessite au moins une autre irradiation de l'objet, qui reçoit donc une dose beaucoup plus importante, nuisible dans le cas courant d'examen d'êtres vivants.

Une autre façon de procéder consiste à disposer deux détecteurs l'un derrière l'autre, dont le premier reçoit le rayonnement complet et le second le rayonnement primaire seul, grâce à des collimateurs qui arrêtent le rayonnement diffusé. Chaque acquisition d'une image bidimensionnelle par le détecteur est faite en une seule fois contrairement au procédé précédent, de sorte qu'aucune augmentation de dose d'irradiation n'est subie par l'objet. On estime le rayonnement primaire sur le second détecteur en le mesurant aux endroits qui sont alignés avec une ouverture de collimateur et la source du rayonnement, et en appliquant une correction nécessitée par l'absorption du rayonnement dans le premier détecteur. Une soustraction des mesures du premier détecteur par le rayonnement primaire estimé par le second détecteur aux mêmes endroits y donne une estimation du rayonnement diffusé ; des interpolations entre ces endroits complètent encore une image bidimensionnelle du rayonnement diffusé. L'inconvénient de cette façon de procéder est que l'installation est coûteuse à cause des deux détecteurs.

Dans une autre catégorie de procédés, on utilise des calibrations pour estimer le rayonnement diffusé. Des images sont prises en utilisant, à la place de l'objet à étudier, des plaques ou d'autres pièces de forme simple, souvent appelées fantômes de calibration, construites de préférence en des matières ayant des propriétés d'absorption et de diffusion analogues à celle de l'objet. On utilise aussi le réseau de petites billes du premier procédé pour l'appliquer aux fantômes de calibration et obtenir successivement des images du rayonnement primaire et du rayonnement diffusé pour chaque fantôme de calibration. Les mesures faites avec l'objet sont corrigées par une fonctionnelle en utilisant un noyau de convolution défini par les résultats de calibration, en utilisant de préférence les images d'un fantôme choisi, supposé ressembler le plus à l'objet en raison d'une similitude de leurs mesures. Ce procédé est assez simple mais implique des approximations importantes. Un exemple est donné dans le document US 2005/0078787 A1.

Dans une autre famille de procédé, on travaille sur des voxels de l'objet et non sur des projections du rayonnement. On peut citer une méthode de Monte Carlo par itérations où, après la reconstruction d'une première image tridimensionnelle de l'objet en utilisant les mesures brutes, le rayonnement diffusé correspondant est simulé, puis soustrait de chacune des mesures pour obtenir une première estimation des projections de rayonnement primaire. Une deuxième image tridimensionnelle de l'objet est reconstruite, et son rayonnement diffusé est de nouveau simulé et soustrait des mesures. Les images tridimensionnelles convergent vers l'image réelle de l'objet en répétant ce procédé, mais les temps de calcul sont excessifs.

Dans un autre procédé de cette seconde famille, le rayonnement diffusé par chacun des voxels d'une première image tridimensionnelle de l'objet est évalué par une méthode analytique, puis corrigé par un facteur, et une deuxième image de l'objet est encore construite en soustrayant le rayonnement diffusé estimé des mesures. Contrairement au procédé précédent, aucune nouvelle image de l'objet n'est recherchée, le traitement analytique étant supposé donner une convergence immédiate ; mais le temps de calcul reste important pour des résultats moins bons que ceux du procédé précédent. Un exemple de ce procédé est donné par le document US 2005/0185753 A1.

L'invention proposée ici implique une combinaison originale, comprenant à la fois l'application d'une fonctionnelle de convolution typique des méthodes d'analyse projection par projection, tout en recourant temporairement à une décomposition de l'objet en voxels, d'une façon qui permet à la fois d'estimer le rayonnement diffusé avec une meilleure précision que les méthodes utilisant les projections sans nécessiter les temps de calcul importants associés aux méthodes où l'objet est décomposé en voxels.

Sous sa forme la plus générale, l'invention est relative à un procédé d'estimation d'un rayonnement diffusé par un objet étudié en utilisant un rayonnement traversant l'objet et un détecteur bidimensionnel recueillant le rayonnement après qu'il a traversé l'objet, comprenant des prises d'images de l'objet par le détecteur en mesurant un rayonnement total, somme du rayonnement diffusé et d'un rayonnement primaire ayant traversé l'objet sur des projections rectilignes ; et aussi des prises d'images de fantômes de calibration traversés successivement par le rayonnement en mesurant encore pour chaque fantôme un rayonnement diffusé par le fantôme et en rayonnement total, somme du rayonnement diffusé par le fantôme et d'un rayonnement primaire ayant traversé le fantôme sur des projections rectilignes ; comprenant encore des étapes : de formation d'une image tridimensionnelle de l'objet à partir des images de l'objet prises par le détecteur ; d'attribution de coefficients respectifs de diffusion du rayonnement à chaque portion de l'image tridimensionnelle ; de détermination, pour les projections du rayonnement à travers l'objet, d'une image de longueurs équivalentes de matériau des fantômes quant à une quantité totale de rayonnement diffusé sur les projections ; de choix du fantôme d'après une similitude de l'image de longueurs équivalente et d'une épaisseur traversée par le rayonnement de mesure dudit fantôme choisi ; et d'extrapolation du rayonnement diffusé par objet du rayonnement diffusé par le fantôme choisi.

De façon favorable, l'extrapolation est faite en appliquant, à chacune des projections, des fonctionnelles au rayonnement total de l'objet et au rayonnement total du fantôme choisi, pour calculer une diffusion du rayonnement par un calcul ; et le procédé comprend encore des étapes consistant à faire un rapport numérique de la fonctionnelle sur l'objet et de la fonctionnelle sur le fantôme choisi, et à multiplier le rayonnement diffusé par le fantôme choisi par le rapport numérique, pour chacune des projections.

Dans une réalisation préférée, les fonctionnelles sont de la forme F = ($\phi_{acquis}$ x L)*K, où F est le rayonnement diffusé simulé, $\phi_{acquis}$ est le rayonnement total, x est un opérateur de multiplication, L est l'image des longueurs équivalentes de l'objet ou la longueur équivalente du fantôme choisi traversée par la projection, * est un opérateur de convolution bidimensionnelle et K est un noyau de distribution bidimensionnelle du rayonnement diffusé par la projection.

L'invention sera décrite maintenant en référence aux figures qui en illustrent certaines réalisations et que voici :

- la figure 1 illustre l'acquisition de mesures sur l'objet à étudier,
- la figure 2 illustre le mode de calibration,
- la figure 3a et 3b donnent la représentation d'un objet effectivement étudié en vues de dessus et de côté,
- la figure 4a, 4b, 4c et 4d représentent les résultats des reconstructions obtenues à partir de projections de mesures brutes et de projections corrigées en soustrayant le rayonnement diffusé par cet objet, estimé de trois façons différentes,
- et la figure 5 est un organigramme du procédé.

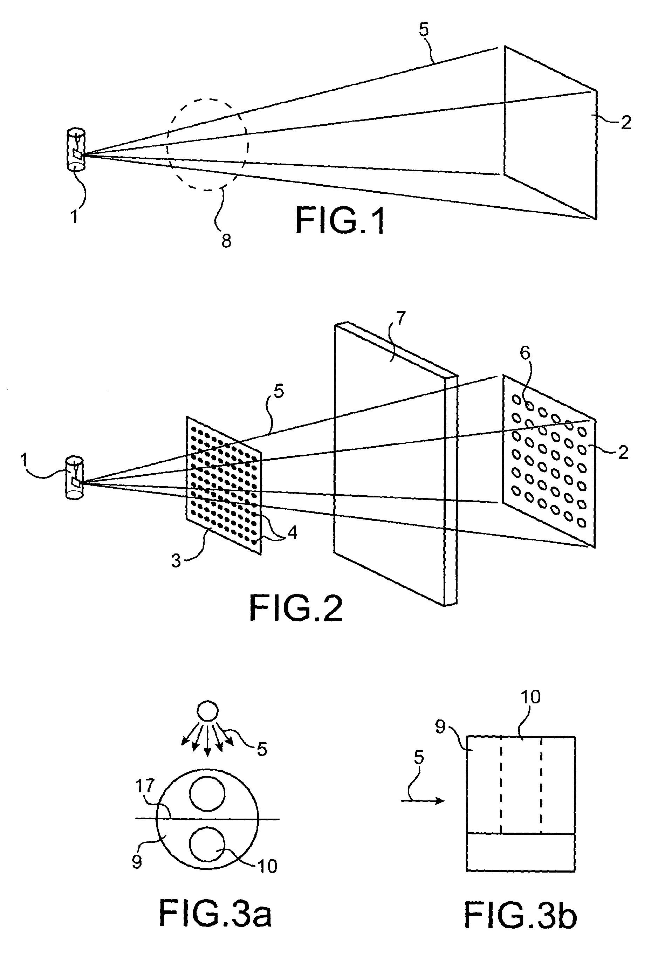

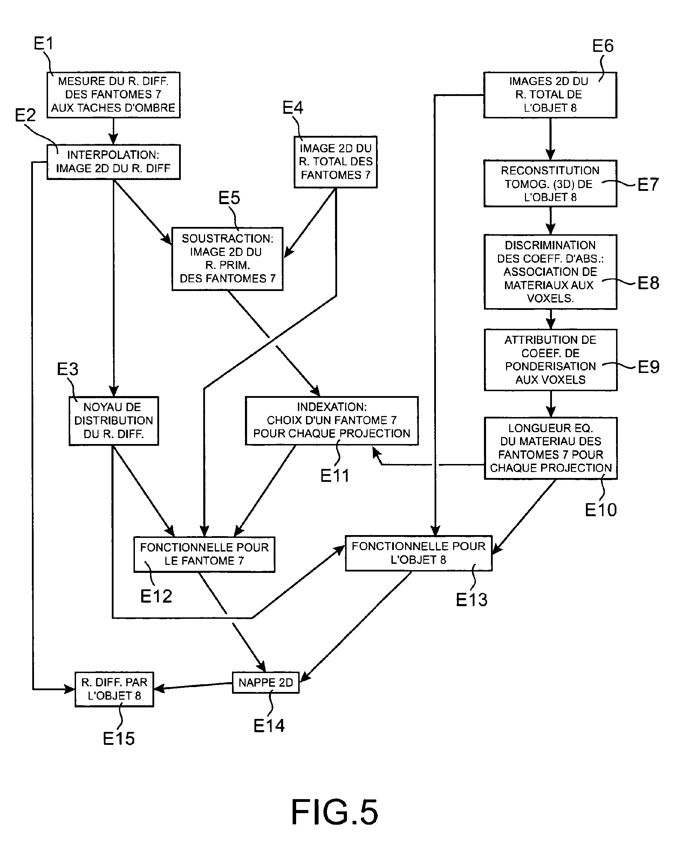

La figure 2 montre que l'appareillage comprend une source 1 et un détecteur bidimensionnel 2. Un objet 8 à étudier est placé entre eux et atténue un rayonnement 5 conique émis par la source 1 avant qu'il n'arrive au détecteur 2. Le rayonnement 5 traverse l'objet 8 par des projections rectilignes. Le détecteur 2 mesure l'atténuation du rayonnement sur chacune des projections. Une image tridimensionnelle de l'objet peut être obtenue en faisant tourner ensemble la source 1 et le détecteur 2 autour de lui et en prenant plusieurs images bidimensionnelles par le détecteur 2 à des incidences différentes, dont la synthèse est faite. Dans une étape préliminaire du procédé illustrée à la figure 1, correspondant à une calibration, l'objet 8 est cependant absent et remplacé dans la même installation par une série de fantômes 7 dont l'un d'entre eux est illustré et qui sont étudiés successivement après avoir été placés dans le rayonnement 5. Il s'agit de plaques ou d'autres structures simples construites en un matériau homogène, dont les propriétés de diffusion et d'absorption du rayonnement sont de préférence analogues à celles d'un des matériaux devant constituer l'objet 8 afin d'avoir une bonne similitude des comportements. Un absorbeur 3 est aussi placé dans le rayonnement 5 ; il est composé d'éléments absorbeurs tels que des billes 4 séparées de distances connues. Le rayonnement 5 se projette sur le détecteur 2 avec des taches d'ombre 6 correspondant aux projections des billes 4 et où on mesure, en l'absence de rayonnement primaire ayant traversé le fantôme 7 d'un trajet rectiligne et qui a été complètement absorbé par les billes 4, le rayonnement diffusé par le fantôme 7 à ces endroits (étape E1 de la figure 5). Une image complète du rayonnement diffusé est obtenue en interpolant les mesures faites aux taches d'ombre 6 sur le reste de l'étendue du détecteur 2 (étape E2). Un noyau de distribution du rayonnement diffusé peut être déduit d'autres mesures faites sur ce fantôme 7 (étape E3), éventuellement au moyen d'autres techniques connues dans l'art. Ce noyau de distribution exprime l'étalement du rayonnement diffusé le long d'une projection sur la région environnante de l'image. On peut souvent le modéliser par une fonction gaussienne bidimensionnelle en fonction des rayons séparant le point d'arrivée de la projection et chacun des points de la régions environnante, et qui dépend alors d'un seul paramètre : l'écart-type.

Une autre mesure du fantôme 7, l'absorbeur 3 étant cette fois enlevé, permet d'en obtenir une autre image, qui exprime la somme du rayonnement primaire et du rayonnement diffusé à chaque pixel du détecteur 2 (étape E4). La soustraction des deux images à chaque pixel du détecteur 2 donne ensuite une image du rayonnement primaire seul (E5). Les images du rayonnement primaire, du rayonnement diffusé et le noyau de distribution restent associés au fantôme 7. Cela est répété pour tous les fantômes 7 de façon à obtenir un catalogue de calibration.

Les étapes suivantes du procédé concernent l'objet 8. Une série d'images bidimensionnelles en est prise dans la configuration de la figure 1 à plusieurs incidences (étape E6). Une première reconstitution tomographique, tridimensionnelle de l'objet 8 en voxels est obtenue en utilisant un procédé d'inversion quelconque de l'art comme l'algorithme de Feldkamp (étape E7). Chaque voxel est exprimé par le coefficient d'atténuation qu'il est estimé faire subir au rayonnement, sans tenir compte de ce que le rayonnement diffusé a troublé les mesures. Les matériaux constitutifs de l'objet 8, supposés connus au préalable, sont respectivement associés aux voxels en appliquant des critères de seuil d'après les valeurs connues des coefficients d'atténuation de ces matériaux constitutifs et ceux que le procédé d'inversion a calculé aux voxels (étape E8). Des coefficients de pondération "p", égaux aux rapports de la section efficace de diffusion Compton des matériaux sur celle du matériau de calibrage (des fantômes 7), sont attribués aux voxels d'après le matériau qui a été associé à chacun (étape E9). Pour des applications en anatomie, on pourra retenir l'air, les tissus mous et les os comme matériaux constitutifs.

Une image bidimensionnelle d'une longueur traversée équivalente, $L_{equ}$, est ensuite obtenue à l'aide de l'image tridimensionnelle des coefficients de pondération "p" en appliquant la formule :

$$L_{equ}(i) = \int_{l=0}^{L(i)} p(l) \times dl \, ,$$

où $L(i)$ est l'épaisseur réelle de l'objet traversée par le rayon primaire qui atteint le détecteur au pixel noté i (étape E10). $L_{equ}$ correspond à la longueur que chaque rayon du rayonnement 5 devrait traverser dans le matériau des fantômes 7 pour donner le même rayonnement diffusé qu'à travers son parcours dans l'objet 8. Quand $L_{equ}$ de chaque projection est obtenue, le fantôme 7 ayant l'épaisseur la plus proche de la moyenne de $L_{equ}$ sur une zone d'indexation, est indexé à la projection (étape E11). En variante, un fantôme fictif ayant une épaisseur égale à $L_{equ}$ et dont les propriétés seraient obtenues par interpolation entre deux fantômes 7 réels, mesurés, pourrait être indexé à la projection.

Une fonctionnelle F, qui donne le taux de diffusion Compton du premier ordre du rayonnement, est ensuite appliquée à chaque projection mesurée du fantôme 7 indexé (étape E12) et de l'objet 8 (étape E13). Elle est définie comme :

$$F = \left( \Phi_{acquis} \times L_{equ} \right) * K$$

pour l'objet 8, où L représente l'image des longueurs équivalentes calculées sur l'objet, $\phi_{acquis}$ le rayonnement total parvenu au détecteur 2 pour la projection considérée, et K le noyau de distribution du rayonnement diffusé. La même fonctionnelle est appliquée au fantôme 7 indexé en remplaçant $L_{equ}$ par l'épaisseur de celui-ci. L'application de la fonctionnelle donne une simulation du pouvoir de diffusion du rayonnement.

Le rapport de la fonctionnelle F de l'objet 8 sur celle du fantôme 7 est appelé nappe et noté $\alpha$ (obtenu à l'étape E14). Il est défini pour chacune des projections et donne le rayonnement diffusé relatif de l'objet 8 par rapport à celui du fantôme 7. La nappe $\alpha$ est ensuite multipliée par le diffusé du fantôme 7, tel qu'il a été obtenu au moyen de l'absorbeur 3 et des interpolations des valeurs mesurées aux taches d'ombre 6, pour obtenir une estimation du rayonnement diffusé de l'objet 8 sur chacune des projections (étape E15).

L'image du diffusé D de l'objet 8 est ainsi estimée par la formule suivante, appliquée à chacune des projections :

$$D = D_{calib} \times \left( \left( \frac{\Phi_{acquis} \times L_{equ}}{\Phi_{calib} \times L_{calib}} \right) * K \right) ,$$

où $D_{calib}$ est l'image du rayonnement diffusé associée au fantôme 7 d'épaisseur équivalente à l'objet 8, $\phi_{acquis}$ et $\phi_{calib}$ sont les projections du rayonnement total reçu pour l'objet 8 et le fantôme 7, $L_{equ}$ et $L_{calib}$ sont l'image de longueurs équivalentes de l'objet 8 traversé par le rayonnement 5 et l'épaisseur du fantôme 7, et K est le noyau de distribution du rayonnement calibré.

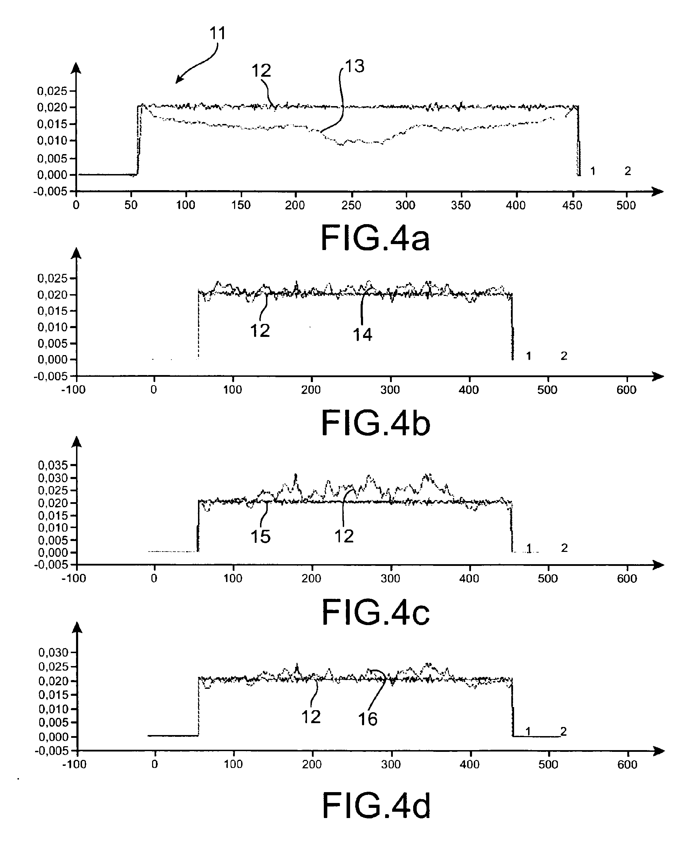

Des résultats d'essais sont donnés au moyen des figures suivantes. Les figures 3a et 3b représentent un objet composé d'un cylindre 9 de Plexiglas et muni de deux perçages 10 coaxiaux d'air et pouvant donc être comparé, pour l'absorption du rayonnement, à un thorax et à ses deux poumons. Les figures 4a, 4b, 4c et 4d représentent des résultats d'atténuation du rayonnement à travers le cylindre 9, de reconstructions de coefficients à partir de projections mesurées dans toutes les directions par un rayonnement 5 occupant nécessairement plusieurs positions d'incidence autour du cylindre 9. La reconstruction était faite sur une ligne 17 diamétrale du cylindre 9 donnée sur la courbe 11 commune à toutes les figures 4a et 4b passant entre les perçages 10 et donc se traduit par un plateau 12 correspondant à un matériau homogène. L'objet est homogène donc le profil coefficients d'atténuation attendu est un plateau par un rayonnement 5. La figure 4a représente les coefficients d'atténuations bruts 13, obtenus à partir des projections mesurées, sans corriger l'influence du rayonnement diffusé : le plateau 12 est remplacé par une forme d'auge. A la figure 4b, on donne les résultats obtenus en faisant une seconde mesure en utilisant l'absorbeur 3 pour évaluer le rayonnement diffusé puis le soustraire du rayonnement total (courbe 14) : on voit que le rayonnement diffusé est bien évalué, les coefficients d'atténuation estimés étant partout presque semblables aux coefficients d'atténuation réels, mais au prix d'une irradiation accrue ainsi qu'on l'a signalé.

A la figure 4c, on donne le résultat obtenu selon le procédé, commenté plus haut, de US 2005/0078787 A1 (courbe 15) : le résultat est meilleur que celui de la figure 4a, mais il n'est pas tout à fait satisfaisant, le rayonnement diffusé étant en général surestimé et des variations des coefficients d'atténuation reconstruits étant présentes dans cet exemple.

La figure 4d illustre enfin l'utilisation de l'invention (courbe 16) : les résultats sont aussi bons que dans le cas de la figure 4b, et sans augmentation de la dose reçue.

## Revendications

1. Procédé d'estimation d'un rayonnement diffusé (D) par un objet (8) étudié en utilisant un rayonnement (5) traversant l'objet et un détecteur (2) bidimensionnel recueillant le rayonnement après qu'il a traversé l'objet, comprenant : des prises d'images de l'objet par le détecteur en mesurant un rayonnement total ($\phi_{acquis}$) somme du rayonnement diffusé et d'un rayonnement primaire ayant traversé l'objet sur des projections rectilignes ; et aussi des prises d'images de fantômes (7) de calibration traversés successivement par le rayonnement (5) en mesurant encore pour chaque fantôme un rayonnement diffusé par le fantôme ($D_{calib}$) et un rayonnement total ($\phi_{calib}$), somme du rayonnement diffusé par le fantôme et d'un rayonnement primaire ayant traversé le fantôme sur des projections rectilignes ; comprenant encore des étapes : de formation d'une image tridimensionnelle de l'objet (8) à partir des images de l'objet prises par le détecteur (2) ; d'attribution de coefficients respectifs de diffusion du rayonnement à chaque portion de l'image tridimensionnelle ; de détermination, pour les projections du rayonnement à travers l'objet, d'une image de longueurs équivalentes ($L_{equ}$) de matériau des fantômes quant à une quantité totale de rayonnement diffusé sur les projections ; de choix du fantôme d'après une similitude de la longueur équivalente et d'une épaisseur traversée par le rayonnement de mesure (5) dudit fantôme choisi ; et d'extrapolation de rayonnement diffusé par l'objet (D) du rayonnement diffusé par le fantôme ($D_{calib}$) choisi.

2. Procédé d'estimation d'un rayonnement diffusé par un objet selon la revendication 1, **caractérisé en ce qu'**un des fantômes est choisi indépendamment pour chacune des projections.

3. Procédé d'estimation d'un rayonnement diffusé par un objet selon l'une quelconque des revendications 1 ou 2, **caractérisé en ce que** l'extrapolation est faite en appliquant à chacune des projection des fonctionnelles (F) au rayonnement total de l'objet ($\phi_{aquis}$) et au rayonnement total du fantôme choisi ($\phi_{calib}$), pour calculer une diffusion du rayonnement par une simulation, et **en ce que** le procédé comprend encore des étapes consistant à faire un rapport numérique de la fonctionnelle sur l'objet et de la fonctionnelle sur le fantôme choisi, et à multiplier le rayonnement diffusé par le fantôme choisi ($D_{calib}$) par le rapport numérique, pour chacune des projections.

4. Procédé d'estimation d'un rayonnement diffusé par un objet selon l'une quelconque des revendications 1 ou 2, où l'objet est un être vivant, **caractérisé en ce que** les coefficients de diffusion des portions de l'image tridimensionnelle sont déterminés en attribuant des compositions d'air, de tissus mous ou d'os auxdites portions.

5. Procédé d'estimation d'un rayonnement diffusé par un objet selon la revendication 3, **caractérisé en ce que** les fonctionnelles sont de la forme F = ($\phi_{acquis}$ x L)*K, où F est le rayonnement diffusé simulé, $\phi_{acquis}$ est le rayonnement total, x est un opérateur de multiplication, L est l'image de longueurs équivalentes de l'objet ou la longueur du fantôme choisi traversée par la projection, * est un opérateur de convolution bidimensionnelle et K est un noyau de distribution bidimensionnelle du rayonnement diffusé par la projection.

## Claims

1. Method for estimating the radiation (D) scattered by an object (8) studied using radiation (5) traversing the object and a two-dimensional detector (2) receiving the radiation after it has traversed the object, including: image sensing of the object by the detector, by measuring a total radiation ($\phi_{acquired}$), which is the sum of the scattered radiation and a primary radiation that has traversed the object on rectilinear projections; and also image sensing of calibration phantoms (7) successively traversed by the radiation (5), by measuring, again, for each phantom, a radiation scattered by the phantom ($D_{calib}$) and a total radiation ($\phi_{calib}$), which is the sum of the radiation scattered by the phantom and a primary radiation that has traversed the phantom on rectilinear projections; also including the steps of: forming a three-dimensional image of the object (8) based on images of the object taken by the detector (2); attributing respective scattering coefficients of the radiation at each portion of the three-dimensional image; determining, for the projections of the radiation through the object, an image of equivalent lengths ($L_{equ}$) of material of the phantoms with regard to a total amount of scattered radiation on the projections; and choosing the phantom according to a similarity of the equivalent length and a thickness traversed by the radiation (5) for measuring said chosen phantom; and extrapolating the radiation scattered by the object from the radiation scattered ($D_{calib}$) by the chosen phantom.

**2.** Method for estimating a radiation scattered by an object according to claim 1, **characterised in that** one of the phantoms is chosen independently for each of the projections.

**3.** Method for estimating a radiation scattered by an object according to either one of claims 1 or 2, **characterised in that** the extrapolation is done by applying, on each of the projections, functionals (F) to the total radiation of the object ($\phi_{acquired}$) and to the total radiation of the chosen phantom ($\phi_{calib}$), in order to calculate a scattering of the radiation by a simulation, and **in that** the method also includes steps consisting of obtaining a numerical ratio of the functional on the object and the functional on the chosen phantom, and multiplying the radiation scattered by the chosen phantom ($D_{calib}$) by the numeric ratio, for each of the projections.

**4.** Method for estimating a radiation scattered by an object according to either one of claims 1 or 2, in which the object is a live being, **characterised in that** the scattering coefficients of the three-dimensional image portions are determined by attributing compositions of air, soft tissue or bone to said portions.

**5.** Method for estimating a radiation, scattered by an object according to claim 3, **characterised in that** the functionals are in the form F = ($\phi_{acquired}$ x L)*K, where F is the simulated scattered radiation, $\phi_{acquired}$ is the total radiation, x is a multiplication operator, L is the image of the equivalent lengths of the object or the equivalent length of the chosen phantom through which the projection passes, * is a two-dimensional convolution operator and K is a kernel for a two-dimensional distribution of the radiation scattered by the projection.

**Patentansprüche**

**1.** Verfahren zum Schätzen einer Strahlung (D), welche von einem Objekt (8) gestreut wird, das durch Verwendung einer das Objekt durchquerenden Strahlung (5) und eines zweidimensionalen Detektors (2) untersucht wird, welcher die Strahlung empfängt, nachdem sie das Objekt durchquert hat, umfassend: Aufnehmen von Bildern des Objektes durch den Detektor durch Messen einer Gesamtstrahlung ($\Phi_{empfangen}$) als Summe der gestreuten Strahlung und einer primären Strahlung, welche das Objekt auf geradlinigen Projektionen durchquert hat; und ebenso Aufnehmen von Bildern von Kalibrierungsphantomen (7), welche nacheinander durch die Strahlung (5) durchquert werden, wobei für jedes Phantom noch eine von dem Phantom gestreute Strahlung ($D_{kalib}$) und eine Gesamtstrahlung ($\Phi_{kalib}$) gemessen werden, welche die Summe der von dem Phantom gestreuten Strahlung und einer primären Strahlung ist, welche das Phantom auf geradlinigen Projektionen durchquert hat, ferner umfassend die Schritte: Bilden eines dreidimensionalen Bildes des Objekts (8) ausgehend von den von dem Detektor (2) aufgenommenen Bildern des Objektes; Zuweisung von jeweiligen Streukoeffizienten der Strahlung zu jedem Abschnitt des dreidimensionalen Bildes; Bestimmen eines Bildes mit äquivalenten Materiallängen ($L_{äqu}$) der Phantome in Bezug auf eine Gesamtmenge von auf den Projektionen gestreuter Strahlung für die Projektionen der Strahlung durch das Objekt hindurch; Auswahl des Phantoms gemäß einer Ähnlichkeit der äquivalenten Länge und einer Dicke, welche von der Strahlung (5) zum Messen des gewählten Phantoms durchquert wird; und Extrapolieren von von dem Objekt gestreuter Strahlung (D) aus der von dem gewählten Phantom gestreuten Strahlung ($D_{kalib}$).

**2.** Verfahren zum Schätzen einer von einem Objekt gestreuten Strahlung nach Anspruch 1, **dadurch gekennzeichnet, dass** eines der Phantome unabhängig für jede der Projektionen gewählt wird.

**3.** Verfahren zum Schätzen einer von einem Objekt gestreuten Strahlung nach einem der Ansprüche 1 oder 2, **dadurch gekennzeichnet, dass** die Extrapolation durch Anwenden von Funktionalen (F) von der Gesamtstrahlung des Objekts ($\Phi_{empfangen}$) und von der Gesamtstrahlung des gewählten Phantoms ($\Phi_{kalib}$) auf jede der Projektionen durchgeführt wird, um eine Streuung der Strahlung durch eine Simulation zu berechnen, und dass das Verfahren ferner Schritte umfasst, die bestehen in:

Bilden eines numerischen Verhältnisses des Funktionals auf dem Objekt und des Funktionals auf dem gewählten Phantom und Multiplizieren der von dem gewählten Phantom gestreuten Strahlung ($D_{kalib}$) mit dem numerischen Verhältnis für jede der Projektionen.

4. Verfahren zum Schätzen einer von einem Objekt gestreuten Strahlung nach einem der Ansprüche 1 oder 2, wobei das Objekt ein Lebewesen ist, **dadurch gekennzeichnet, dass** die Streukoeffizienten der Abschnitte des dreidimensionalen Bildes bestimmt werden durch Zuweisen von Zusammensetzungen von Luft, weichem Gewebe oder Knochen zu diesen Abschnitten.

5. Verfahren zum Schätzen einer von einem Objekt gestreuten Strahlung nach Anspruch 3, **dadurch gekennzeichnet, dass** die Funktionale von der Form $F = (\Phi_{empfangen} \times L) * K$ sind, wobei F die simulierte gestreute Strahlung ist, $\Phi_{empfangen}$ die Gesamtstrahlung ist, x ein Multiplikationsoperator ist, L das Bild äquivalenter Längen des Objekts oder die von der Projektion durchquerte Länge des gewählten Phantoms ist, * ein Operator einer zweidimensionalen Konvolution ist und K ein Kern einer zweidimensionalen Verteilung der von der Projektion gestreuten Strahlung ist.

FIG.1

FIG.2

FIG.3a

FIG.3b

FIG.4a

FIG.4b

FIG.4c

FIG.4d

FIG.5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20050078787 A1

- US 20050185753 A1